# EUROPÄISCHE PATENTANMELDUNG

(11) **EP 0 760 481 A1**
(43) Veröffentlichungstag der Anmeldung: **05.03.1997**
(21) Anmeldenummer: 95113601.9
(22) Anmeldetag: 30.08.1995
(51) Int. Cl.: G01N 33/28, F01M 11/10

(54) **Vorrichtung zur Kontrolle des Motoröl-Alterungsgrades für ein Fahrzeug**

(71) Anmelder: Park, Kyu Young, Yongsan-Ku, Seoul (KR)
(72) Erfinder: Yang, Se Hoon, Anyang, Kyungki-Do (KR)
(74) Vertreter: COHAUSZ HASE DAWIDOWICZ & PARTNER

(57) **Zusammenfassung**

Motoröl-Kontrollvorrichtung für ein Fahrzeug, mit dem sich der Alterungsgrad des Motoröls ohne zusätzliche Maßnahmen prüfen läßt und sich der Fahrer damit jederzeit über den Alterungsgrad des Motoröls informiern kann, ausgestattet mit einem Konstantstromgenerator zur Erzeugung eines Stroms von definierter Stromstärke, einem mit diesem Konstantstromgenerator verbundenen Detektor zur Einstrahlung von Licht in das Motoröl und Erfassung des das Öl durchdringenden Lichtanteils, einem Stromveränderungsdetektor zur Erfassung von Änderungen des Ausgangs des Detektors, einer Signalverarbeitungseinheit zur Verarbeitung von Signalen, die der Größe der von dem Stromveränderungsdetektor erfaßten Stromstärkenänderung entsprechen, sowie einem Anzeigeinstrument zur Anzeige der von der Signalverarbeitungseinheit verarbeiteten Signale, so daß der Fahrer den Ölwechselzeitpunkt problemlos feststellen kann und damit die anfallende Altölmenge aus Fahrzeugmotoren in vorteilhafter Weise verringert wird.

## Beschreibung

### Hintergrund der Erfindung

### 1. Gebiet der Erfindung

Die Erfindung betrifft eine Vorrichtung zur Kontrolle des Motoröl-Alterungsgrades für ein Fahrzeug, insbesondere eine Alterungsgrad-Kontrollvorrichtung für Fahrzeug-Motoröle, die den jeweiligen Alterungsgrad des Motoröls mit Hilfe eines Photosensors ermittelt und ihn auf einer Anzeigevorrichtung darstellt, um auf diese Weise den rechtzeitigen Zeitpunkt für einen Motorölwechsel zu melden.

### 2. Beschreibung des bisherigen Standes der Technik

Das Schmieröl in Fahrzeugmotoren dient im wesentlichen zu dem Zweck, die Reibungskräfte zwischen den Motorteilen zu verringern und damit das zwischen diesen Teilen erzielte mechanische Leistungsverhalten zu optimieren. Ein Motoröl dieser Art benötigt eine bestimmte Viskosität sowie einen gewissen natürlichen Reinheitsgrad. Mit zunehmender Nutzung des Öls tritt jedoch eine Verringerung seiner Viskosität sowie ein unvorteilhaftes Nachlassen der natürlichen Ölreinheit ein, wodurch sich die Gefahr von Abrieb zwischen den einzelnen Motorteilen ergibt. Bei Fahrzeugen werden aus diesem Grund in bestimmten Abständen Ölwechsel erforderlich. Dabei unterscheiden sich die Ölwechselintervalle je nach Art und Alter des Fahrzeugs. Bei Personenkraftwagen werden Ölwechsel jeweils nach etwa 4000 km Gesamtfahrleistung fällig, und zwar unabhängig vom Alterungsgrad des Öls. Wird der Zeitpunkt des Ölwechsels zu lange herausgezögert, kann sich die Lebensdauer des Fahrzeugs infolge der Beeinträchtigung des mechanischen Kennverhaltens verkürzen. Zu frühe Ölwechsel sind dagegen teuer und unter dem Gesichtspunkt der überflüssigen Altölentsorgung bedenklich. Aus den genannten Gründen ist es wünschenswert, den Ölwechsel jeweils zum optimalen Zeitpunkt durchzuführen.

Der Zeitpunkt des Motorölwechsels wird, wie eingangs erläutert, durch den Fahrer anhand der Fahrleistung oder einer Sichtprüfung der Ölviskosität bestimmt. Da dies jedoch ohne geeignete Bezugswerte und nicht anhand eines standardisierten Verfahrens erfolgt und die Kontrolle des Ölalterungsgrades somit nur auf der Erfahrung des Fahrers basiert, wird der optimale Ölwechselzeitpunkt mitunter nicht festgestellt, wodurch sich erhöhte Kosten sowie unerwünschte Umweltverschmutzungsgefahren ergeben.

Benötigt ein Kraftfahrzeug z.B. zum Ölwechsel vier Liter Motoröl und hat bei einem vorgeschriebenen Ölwechselintervall von 4.000 km eine Jahreslaufleistung von 20.000 km, so beläuft sich der Ölverbrauch dieses Fahrzeugs auf 20 l jährlich. Nimmt man an, daß in einem gegebenen Land insgesamt 10 Millionen Fahrzeuge zugelassen sind, so liegt der Motoröl-Gesamtverbrauch dieses Landes bei 200 Millionen Liter pro Jahr.

Durch die Durchführung des Motorölwechsels zu einem präzise ermittelten Zeitpunkt - d.h. die Möglichkeit, sich anstatt nach einer Laufleistung von 4000 km erst nach 4500 km für einen Ölwechsel zu entscheiden und damit die Laufleistung um 500 km zu erhöhen - läßt sich die Menge anfallenden Altöls aus Fahrzeugmotoren um ca. 1/8 verringern. Dies bedeutet im einzelnen eine Reduzierung der Altölmenge aus Fahrzeugmotoren um ca. 30 Millionen Liter und damit eine erhebliche Umweltschutzwirkung. Zudem wird der Ölwechsel für den Fahrer komfortabler handhabbar, da er den genauen Zeitpunkt, an dem das Öl gewechselt werden muß, problemlos feststellen und damit möglichen Schäden an seinem Fahrzeug vorbeugen kann.

### Zusammenfassung der Erfindung

Eine Aufgabe der vorliegenden Erfindung besteht somit darin, eine Motoröl-Kontrollvorrichtung für ein Fahrzeug zu schaffen, die eine Verringerung des Anfalls verbrauchten Motoröls durch Erkennung des rechtzeitigen Motorölwechselzeitpunktes erlaubt.

Eine weitere Aufgabe der vorliegenden Erfindung besteht darin, eine Motoröl-Kontrollvorrichtung für ein Fahrzeug bereitzustellen, mittels derer sich der Alterungsgrad des Motoröls ohne zusätzlichen Handgriff prüfen läßt, d.h. daß der Fahrer den Alterungsgrad des Motoröls jederzeit problemlos kontrollieren kann, wann immer er dies wünscht.

Zur Erfüllung der vorstehenden Aufgaben wird ein Motoröl-Kontrollgerät für ein Fahrzeug vorgestellt, das einen Konstantstromgenerator zur Erzeugung eines Stroms von definierter Stärke, einen mit diesem Konstantstromgenerator verbundenen Detektor zur Einstrahlung von Licht in das Motoröl und Erfassung des das Öl durchdringenden Lichtanteils, einen Stromänderungsdetektor zur Erfassung der Detektoränderung, eine Signalverarbeitungseinheit zur Verarbeitung von Signalen, die der Größe der von dem Stromänderungsdetektor festgestellten Stromänderungen entsprechen, sowie eine Anzeigeeinheit zur Darstellung der von der Signalverarbeitungseinheit verarbeiteten Signale umfaßt.

### Kurzbeschreibung der Zeichnungen

Abb. 1 zeigt den Aufbau der erfindungsgemäßen Vorrichtung zur Prüfung des Motoröl-Alterungsgrades in Form eines schematischen Blockschaltbilds.

Abb. 2 stellt eine perspektivische Ansicht eines Detektors aus Abb. 1 dar.

Abb. 3 stellt eine Prinzipdarstellung einer Anzeigeeinheit aus Abb. 1 gemäß einer weiteren Ausführungsform der Erfindung dar.

### Detaillierte Beschreibung der Erfindung

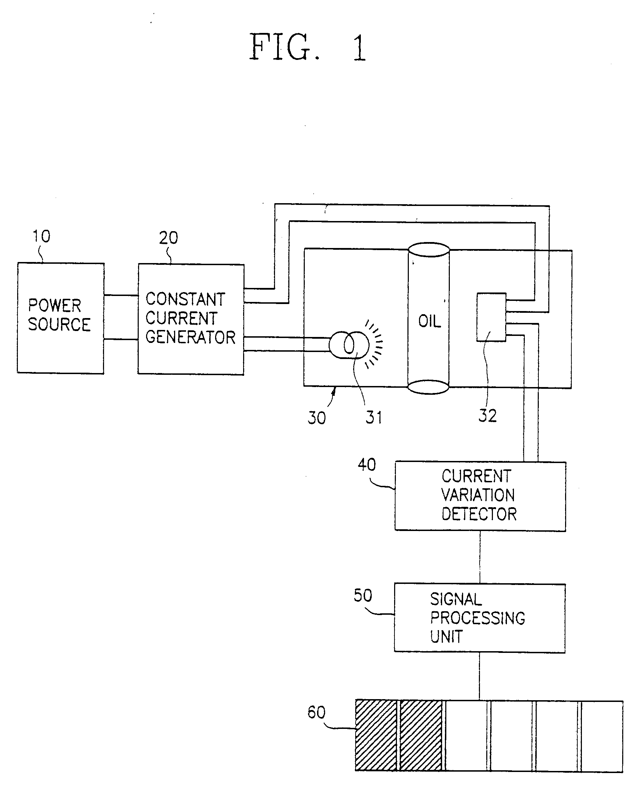

Wie in Abb. 1 verdeutlicht, umfaßt die erfindungsgemäße Vorrichtung zur Kontrolle des Motoröl-Alterungsgrades für ein Fahrzeug einen Konstantstromgenerator 20, der den von einer Stromquelle 10 gelieferten Strom auf konstantem Niveau hält, einen Fotodetektor 30 mit einem Leuchtmittel 31 wie z.B. einer Leuchtdiode (LED), das bei Anliegen des von dem Konstantstromgenerator 20 gelieferten Stroms ein Licht von definierter Intensität abstrahlt, sowie einer Lichtempfangsvorrichtung 32, die in definierten Abstand zu dem Leuchtmittel 31 angeordnet ist und das von diesem Leuchtmittel 31 abgegebene Licht aufnimmt, einen Stromänderungsdetektor 40 zur Erfassung von Änderungen der von der Lichtempfangsvorrichtung 32 abgegebenen Stromstärke, eine Signalverarbeitungseinheit 50 zur Verarbeitung der Signale, die der von dem Stromänderungsdetektor 40 erfaßten Stromänderung entsprechen, sowie ein Anzeiginstrument 60 zur Wiedergabe des Ausgangs der Signalverarbeitungseinheit 50.

Die Spannungsquelle 10 kann ihre Spannung dabei von ihrem eigenen Spannungsversorgungssystem oder von der im Fahrzeug vorhandenen Batterie beziehen.

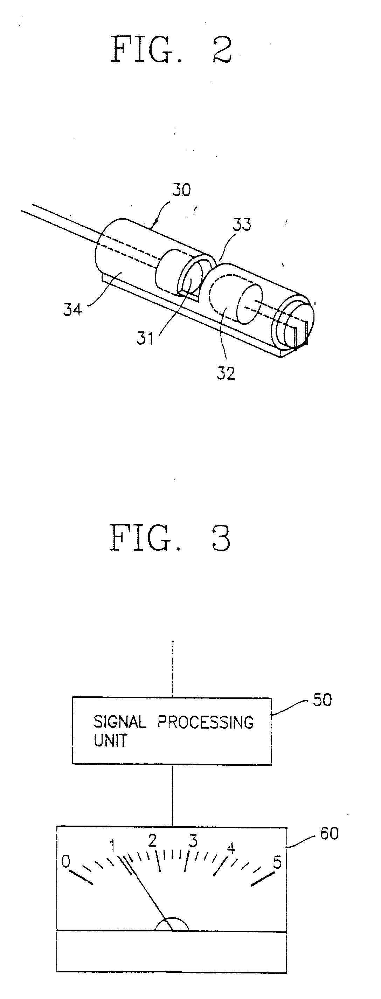

Aus Abb. 2 ist ersichtlich, daß der Fotodetektor 30 ein Gehäuse 34 aufweist, in dessen Mittelteil eine durch einen einseitigen Einschnitt gebildete Ölnut 33 vorgesehen ist, die im wesentlichen der Aufnahme von Motoröl dient, so daß das Motoröl im wesentlichen zu dem Leuchtmittel 31 und der in einem Abstand dazu angeordneten Lichtempfangsvorrichtung 32 gelangen kann. Der Detektor 30 läßt sich dabei an einem beliebigen Ort anordnen, an dem ein ausreichender Ölstrom zur Beaufschlagung des Detektors 30 gewährleistet ist, so daß die Motorölnut 33 von Motoröl durchströmt wird. Dabei läßt sich der Detektor 30 vorzugsweise im Umkreis eines Ölfilters, eines Öltemperatursensors oder eines Teils einer Ölablaßschraube montieren.

Für die Lichtempfangsvorrichtung 32 kommen zudem Elemente wie z.B. ein eindimensionaler Bildsensor, eine CdS-Zelle, eine Photodiode oder eine Solarzelle in Frage, deren Kennverhalten dadurch geprägt ist, daß sich ihr Ausgangssignal in Abhängigkeit von der aufgenommenen Lichtintensität ändert.

Das Anzeiginstrument 60 kann aus einer LED-Balkendiagrammanzeige mit mehrstufiger Skala (wie in Abb. 1) oder aus einem Analoganzeigegerät 60' mit Zeiger bestehen, wie in Abb. 3 dargestellt.

Das Anzeigeinstrument 60 bzw. 60' läßt sich dabei an einem definierten Platz im Fahrzeug montieren, vorzugsweise jedoch in der Instrumententafel eines Armaturenbretts, damit der Fahrer den Alterungsgrad des Motoröls unschwer kontrollieren kann.

Die Funktionsweise der erfindungsgemäßen Vorrichtung zur Kontrolle des Motoröl-Alterungsgrades in einem Fahrzeug wird nachfolgend erläutert.

Der Detektor 30 ist zunächst von Motoröl umgeben, wobei dieses Motoröl den Detektor 30 im wesentlichen ganz beaufschlagt und dabei auch die Motorölnut 33 durchströmt. Das Licht von dem Leuchtmittel 31 wird dabei durch das in der Motorölnut 33 befindliche Motoröl zu der Lichtempfangsvorrichtung 32 übertragen. Weist das Motoröl noch keinen fortgeschrittenen Alterungszustand auf und ist somit noch weitgehend lichtdurchlässig, so fällt nahezu das gesamte von dem Leuchtmittel 31 abgegebene Licht auf die Lichtempfangsvorrichtung 32, so daß der von dieser Lichtempfangsvorrichtung abgegebene Strom nahezu unverändert bleibt und der Stromänderungsdetektor 40 keine Abweichung erfaßt. Auf dem Anzeigeinstrument 60 bzw. 60' erscheint daher keine Anzeige.

Mit zunehmendem Alterungsgrad des Motoröls wird dieses jedoch zunehmend lichtundurchlässig, d.h. es dringt weniger Licht durch das in der Motorölnut 33 befindliche Motoröl. Hierdurch verringert sich der Lichteinfall auf die Lichtempfangsvorrichtung 32, so daß in dieser Vorrichtung eine Änderung der Stromstärke auftritt, die von dem Stromänderungsdetektor 40 erfaßt wird. Da die von dem Stromänderungsdetektor 40 festgestellte Änderung der Stromstärke jedoch relativ geringfügig ausfällt, werden die Signale in der Signalverarbeitungseinheit 50 verstärkt und in eine entsprechende Form gebracht, bevor sie auf dem Anzeigeinstrument 60 bzw. 60' wiedergegeben werden.

Der Fahrer kann den auf dem Anzeigeinstrument 50 angezeigten Alterungsgrad des Motoröls auf diese Weise problemlos kontrollieren und den optimalen Zeitpunkt für einen Motorölwechsel ermitteln.

Wie vorstehend erläutert, läßt sich mit der Vorrichtung zur Kontrolle des Motoröl-Alterungsgrades für ein Fahrzeug der Alterungsgrad des Motoröls derart prüfen, daß der Fahrer den Ölwechselzeitpunkt problemlos feststellen kann und damit die anfallende Altölmenge aus Fahrzeugmotoren in vorteilhafter Weise verringert wird.

Im vorstehenden Text wurde zu Zwecken der Verdeutlichung auf bevorzugte Ausführungsformen der Erfindung Bezug genommen. Für den Fachmann ist jedoch ohne weiteres erkennbar, daß sich die Erfindung auf verschiedene Weise abwandeln, ergänzen oder unter Einsatz alternativer Komponenten realisieren läßt, ohne von Umfang und Wesensart der in den beiliegenden Ansprüchen offengelegten Erfindung abzuweichen.

Dabei läßt sich die Erfindung ebenso wie in einem Fahrzeug insbesondere auch für Industriemaschinen einsetzen, die mit Motoröl arbeiten.

## Patentansprüche

1. Vorrichtung zur Kontrolle des Motoröl-Alterungsgrades für ein Fahrzeug, ausgestattet mit
- einem Konstantstromgenerator (20) zur Erzeugung eines Stroms von definierter Stromstärke;
- einem mit diesem Konstantstromgenerator (20) verbundenen Detektor (30) zur Einstrahlung von Licht in das Motoröl und Erfassung des das Öl durchdringenden Lichtanteils;
- einem Stromänderungsdetektor (40) zur Erfassung von Änderungen des Ausgangs des Detektors (30);
- einer Signalverarbeitungseinheit (50) zur Verarbeitung von Signalen, die der Größe der von dem Stromänderungsdetektor (40) erfaßten Stromstärkenänderung entsprechen;
- einem Anzeigeinstrument (60) zur Anzeige der von der Signalverarbeitungseinheit (50) verarbeiteten Signale.

2. Vorrichtung gemäß Anspruch 1, wobei der Detektor (30) ein Leuchtmittel (31) sowie eine Lichtempfangsvorrichtung (32) umfaßt.

3. Vorrichtung gemäß Anspruch 2, wobei das Leuchtmittel (31) sowie die Lichtempfangsvorrichtung (32) in einem Abstand zueinander auf gegenüberliegenden Seiten einer in einem definierten Teil eines Gehäuses (34) ausgebildeten Ölnut (33) angeordnet und jeweils an diesem Gehäuse befestigt sind.

4. Vorrichtung gemäß Anspruch 1, wobei das Anzeigeinstrument (60) als mehrfach abgestufte Leuchtdioden-Balkendiagrammanzeige ausgeführt ist.

5. Vorrichtung gemäß Anspruch 1, wobei das Anzeigeinstrument als Analogmeßgerät (60') ausgeführt ist.
